# EUROPEAN PATENT SPECIFICATION

(11) **EP 2 647 727 B1**
(45) Date of publication and mention of the grant of the patent: **01.11.2017**
(21) Application number: 13162128.6
(22) Date of filing: 03.04.2013
(51) Int. Cl.: C12R 1/01, B82Y 5/00, C12N 1/20

(54) **Bacterial conductive fibres**
Leitfähige Fasern bakteriellen Ursprungs
Fibres conductrices bactériennes

(30) Priority: 03.04.2012 DK 201270169
(43) Date of publication of application: 09.10.2013
(73) Proprietor: Nielsen, Lars Peter, 8320 Marslet (DK); Meyer, Rikke Louise, 8230 Abyhoj (DK); Boesen, Thomas, 8541 Skodstrup (DK); Sommer, Theis, 0372 Oslo (NO); Song, Jie, 8210 Arhus V (DK); Schramm, Andreas, 8320 Marslet (DK); Pfeffer, Christian, 8300 Odder (DK); Larsen, Steffen, 8200 Arhus N (DK); Pedersen, Niels Risgaard, 8680 Ry (DK); Dong, Mingdong, 8240 Risskov (DK)
(72) Inventor: Nielsen, Lars Peter, 8320 Marslet (DK); Meyer, Rikke Louise, 8230 Abyhoj (DK); Boesen, Thomas, 8541 Skodstrup (DK); Sommer, Theis, 0372 Oslo (NO); Song, Jie, 8210 Arhus V (DK); Schramm, Andreas, 8320 Marslet (DK); Pfeffer, Christian, 8300 Odder (DK); Larsen, Steffen, 8200 Arhus N (DK); Pedersen, Niels Risgaard, 8680 Ry (DK); Dong, Mingdong, 8240 Risskov (DK)
(74) Representative: Høiberg P/S

(56) References cited:
- US-A1- 2012 053 320
- GEMMA REGUERA ET AL: "Extracellular electron transfer via microbial nanowires", NATURE, vol. 435, no. 7045, 23 June 2005 (2005-06-23), pages 1098-1101, XP055037329, ISSN: 0028-0836, DOI: 10.1038/nature03661
- JOSHUA VEAZEY ET AL: "Electronic properties of conductive pili of the metal-reducing bacterium Geobacter sulfurreducens probed by scanning tunneling microscopy", PHYSICAL REVIEW E, vol. 84, no. 6, 1 December 2011 (2011-12-01), pages 1-4, XP055047692, ISSN: 1539-3755, DOI: 10.1103/PhysRevE.84.060901
- D. E. HOLMES ET AL: "Electron Transfer by Desulfobulbus propionicus to Fe(III) and Graphite Electrodes", APPLIED AND ENVIRONMENTAL MICROBIOLOGY, vol. 70, no. 2, 1 February 2004 (2004-02-01), pages 1234-1237, XP055072440, ISSN: 0099-2240, DOI: 10.1128/AEM.70.2.1234-1237.2004
- CHRISTIAN PFEFFER ET AL: "Filamentous bacteria transport electrons over centimetre distances", NATURE, vol. 491, no. 7423, 24 October 2012 (2012-10-24), pages 218-221, XP055072341, ISSN: 0028-0836, DOI: 10.1038/nature11586
- GEMMA REGUERA: "Microbiology: Bacterial power cords", NATURE, vol. 491, no. 7423, 24 October 2012 (2012-10-24), pages 201-202, XP055072360, ISSN: 0028-0836, DOI: 10.1038/nature11638
- LARS PETER NIELSEN ET AL: "Electric currents couple spatially separated biogeochemical processes in marine sediment", NATURE, vol. 463, no. 7284, 25 February 2010 (2010-02-25), pages 1071-1074, XP055072372, ISSN: 0028-0836, DOI: 10.1038/nature08790
- REGINA SCHAUER ET AL: "Succession of cable bacteria and electric currents in marine sediment", THE I S M E JOURNAL: MULTIDISCIPLINARY JOURNAL OF MICROBIAL ECOLOGY, vol. 8, no. 6, 23 January 2014 (2014-01-23), pages 1314-1322, XP055225706, United Kingdom ISSN: 1751-7362, DOI: 10.1038/ismej.2013.239
- NILS RISGAARD-PETERSEN ET AL: "Cable Bacteria in Freshwater Sediments", APPLIED AND ENVIRONMENTAL MICROBIOLOGY, vol. 81, no. 17, 26 June 2015 (2015-06-26) , pages 6003-6011, XP055225712, US ISSN: 0099-2240, DOI: 10.1128/AEM.01064-15
- S. Okabe ET AL: "Sulfate-reducing bacterial community structure and their contribution to carbon mineralization in a wastewater biofilm growing under microaerophilic conditions", Applied Microbiology and Biotechnology, vol. 63, no. 3, 17 July 2003 (2003-07-17), pages 322-334, XP055225709, DE ISSN: 0175-7598, DOI: 10.1007/s00253-003-1395-3

## Description

### Field of invention

The present invention relates to a conductive electronic material based on conductive fibres from bacterial cells belonging to the Desulfobulbaceae family, which comprise a plurality of such conductive fibres and subfibre filaments arranged in parallel in the periphery of a chain of cells.

### Background of invention

Preparation of nanoscale materials, such as nanowires, as electrical conduits between nanocomponents is associated with significant problems in nanoelectronics. Suitable materials must provide sufficient conductivity, and low production costs. The use of protein structures, such as amyloid fibrils, has been proposed which can be readily adsorbed onto a range of suitable substrates. However, the known protein structures typically provide low conductivity levels, which limit their application. Metallization has been considered, but under industrial conditions can be deleterious to protein structure and integrity.

Some bacteria have the ability to assemble small protein subunits, termed pilins, into one or more extracellular structured fibers, pilus or pili. Members of the family Geobacteraceae, for instance, produce pili to facilitate electron transfer to external, solid electron acceptors.

Some microorganisms, such as Shewanella and Geothrix species, may transfer electrons from the cell to the iron oxide surface via excretion of soluble electron-shuttling compounds whereas others, such as Geobacter species, require direct contact with the oxide surface. Previous studies demonstrated that Geobacter metallireducens specifically produced pili during growth on Fe(III) oxide, but not during growth on soluble, chelated Fe(III). Childers, S. E., Ciufo, S. & Lovley, D. R. Geobacter metallireducens accesses insoluble Fe(III) oxide by chemotaxis. Nature 416, 767-769 (2002).

In the prior art (US 2012/053320, G. Reguera et al. Nature, 435, 1098-1101; J. Veazey at al. Physical Review E, 84, 1-4) the pili from bacteria of the *Geobacter* genus was described and, purified pili proteins were assembled to form a protein filament, which is a conductive electronic material; these structures were proposed as nanowires. Bacteria of the *Desulfobulbus* genus had been described which were known to be capable of transferring electrons, thus, they work as a conductive material, like for *Desulfonema* (D. E. Holmes et al., Applied and Environmental Microbiology 70, 1234-1237). The bacteria of *Desulfobulbus* spp, were knonw to form multicellular and filamentous structures (S. Okabe et al. Applied Microbiology and Biotechnology, 63, 322-334).

### Summary of invention

The present invention provides microbial cellular wires and related electronic components and device structures, systems and methods for production and/or assembly, which overcome various deficiencies and shortcomings in the nanoelectronic art. The cellular wires are conductive fibres and/or subfibre filaments, which have been identified within a chain of bacterial cells.

In one aspect, the present invention relates to a conductive electronic material comprising or consisting of one or more conductive fibres and/or subfibre filaments of a chain of bacterial cells, wherein said chain of bacterial cells comprises a plurality of said conductive fibres and/or subfibre filaments arranged in parallel in the periplasmic space along the length and around the periphery of said chain of cells, wherein the outside of said chain of cells is non-conductive, wherein said bacterial cells belong to the Desulfobulbaceae family, and wherein said conductive electronic material comprises or consists of said chain of bacterial cells or is extracted from said chain of bacterial cells. In another aspect, the invention relates to a method of preparing a conductive electronic material consisting of conductive fibres and/or subfibre filaments, said method comprising
a) providing bacterial cells belonging to the Desulfobulbaceae family capable of forming a plurality of parallel conductive fibres and/or subfibre filaments located in the periplasmic space along the length and around the periphery of the cells, wherein the outside of said cells is non-conductive, and
b) incubating said bacterial cells belonging to the Desulfobulbaceae family in a medium conditioned for bacterial proliferation and conductive fibre formation, said medium comprising an electron donor and an electron acceptor, in sufficient time to produce one or more chains of bacterial cells, each chain comprising a plurality of parallel conductive fibres and/or subfibre filaments located in the periplasmic space along the length and around the periphery of said chain of cells.

The invention also in one aspect relates to a use of a chain of bacterial cells belonging to the Desulfobulbaceae family comprising a plurality of parallel conductive fibres and/or subfibre filaments located in the periplasmic space along the length and around the periphery of said chain of cells, wherein the outside of said chain of cells is non-conductive, as a conductive material.

Also, the invention in one aspect provides an electronic device comprising
a) a conductive material of the present invention, or
b) a conductive material produced by a method of the present invention

In a further aspect, the present invention relates to a chain of cells comprising a plurality of parallel conductive fibres and/or subfibre filaments located in the periplasmic space along the length and around the periphery of said chain of cells, wherein the outside of said chain of cells is non-conductive. This application also discloses a cell, such as a bacterial cell belonging to the Desulfobulbaceae family which upon proliferation forms a chain of cells comprising a plurality of parallel conductive fibres and/or subfibre filaments located in the periplasmic space along the length and around the periphery of said chain of cells, wherein the outside of said chain of cells is non-conductive.

The bacterial cell or cells employed in the invention is preferably a bacteria belonging to the Desulfobulbaceae family, such as a bacteria belonging to the Desulfobulbus genus.

### Short Description of Drawings

Figure 1. Demonstration of electric grids in marine sediment.
Figure 2. Observation of the cellular wire.
Figure 3. Abundance of filamentous bacteria in 2 week old "electric" sediment.
Figure 4. Phylogeny
Figure 5. FISH (Fluorescent In Situ Hybridization).
Figure 6. AFM (atomic force microscopy)).
Figure 7. Investigation of the microbial/cellular cable with electrostatic force microscopy (EFM).
Figure 8. TEM (Transmission electron microscopy) image.
Figure 9. Transverse (A) and longitudal (B) TEM view of the bacterial/cellular cables.
Figure 10. Negative stain EM on purified subfibre components

### Detailed description of the invention

### Introduction

The present invention concerns electronic conductive materials comprising or consisting of microbial cellular wires and related electronic components and device structures, systems and methods for production and/or assembly. In particular, the invention concerns protein-based, naturally conductive cellular wires, which can be used as conductive material without resort to metallization techniques or procedures.

The present inventors have found that certain bacteria express a plurality of conductive fibres and subfibre filaments. These cells form chains of cells upon growth, and within such a chain of cells, the conductive fibres and subfibre filaments are arranged in parallel in the periplasmic space along the length and around the periphery of said chain of cells. The periplasmic space is the space between the cytoplasmic membrane and the outer membrane, which is seen in all gram-negative bacteria. Preferably, each cell of the cell chain has its own cytoplasmic membrane, while the outer membrane is shared by all cells in a filament, thus allowing the fibres to be isolated from the surroundings.

The fibres within the cells are conductive, while the outside of the chain of cells is non-conductive. Also, each individual fibre within the cell is conductive inside, but non-conductive on the outside; i.e. the fibres are electrically insulated from other/neighbouring fibres within the cells by invaginations of the outer cell membrane. The cells have been isolated from marine sediment, and it appears that the fact that the cells form long chains of cells allow the cell colony to support electron transfer using the conductive fibres within the cells over longer distances of several centimetres (e.g. 1-15 cm), such as from within an oxygen-deprived marine sediment to the the sediment-water interface. Thus, the fibres are involved in electron transfer reactions across large numbers of cells over relatively long distances of several centimetres. These biologically produced proteinaceous cellular wires could be useful in micro or nanoelectronic applications.

The invention concerns conductive protein-based, nano- or micro-dimensioned wires or conductive components, produced and self-assembled in vitro or in vivo using a native host or engineered microorganisms.

The terms, "conductive fibres" and "cellular wires" are used interchangeably herein. The chain of bacteria describes above comprises conductive fibres and subfibre filaments, which can be used as wires, e.g. in electronic devices, and such wires are referred to as cellular wires.

The disclosure includes one or more systems or methods for the production and self-assembly of protein-based cellular wires, conductive materials, by the use of microorganisms which can be, optionally, genetically modified to express one or a plurality of conductive fibres and/or subfibre filaments arranged in parallel in the periplasmic space along the length and around the periphery of a chain of cells, wherein the outside of said chain of cells is non-conductive.

The invention also provides an electronic device structure and/or a circuit or electrical component thereof comprising at least one conductive fibre, subfibre filament or conductive material of the sort described herein, produced and/or self-assembled in vitro or in vivo using the native host or engineered microorganisms.

Other objects, features, benefits and advantages of the present invention will be apparent from this summary and the following descriptions of certain embodiments, and will be readily apparent to those skilled in the art having knowledge of various parallel conductive fibre-producing microorganisms and similar mechanisms for extracellular electron transfer.

The present invention relates to conductive fibres, and use thereof in various micro or nanotechnological applications. More specifically, without limitation, the present invention also provides for the culture of bacteria belonging to the Desulfobulbaceae family producing a plurality of conductive fibres and/or subfibre filaments arranged in parallel in the periplasmic space along the length and around the periphery of said chain of cells, wherein the outside of said chain of cells is non-conductive. The invention also provides for isolation of such fibres and/or subfibre filaments and, as well as the fabrication of nano-dimensioned electronic circuit components.

Accordingly, this invention is directed to a method of preparing a conductive electronic circuit component. Such a method can comprise providing a bacterium belonging to the Desulfobulbaceae family capable of expressing a conductive proteinaceous fibres and/or subfibre filaments; providing a culture medium conditioned for conductive fibre production, such a medium comprising an electron acceptor; incubating the bacterium and the medium over a time sufficient for fibre and/or subfibre assembly; and isolating conductive fibres and/or subfibre filaments from the medium. In accordance with this invention, conductive fibres and/or subfibre filaments are obtainable from bacterial cells of the present invention. More specifically, without limitation, the gene or genes encoding the conductive proteinaceous fibres and/or subfibre filaments can be isolated and inserted into plasmids or the genome of a recombinant host cell, which cell is then capable of expressing one or more conductive proteinaceous fibres. Such a recombinant microorganism comprising nucleic acid molecules encoding conductive proteinaceous fibres and/or subfibre filaments of the present invention may also be used in the production of a recombinant material. As described more fully below, the conductive fibres and/or subfibre filaments can be produced and/or obtained from systems comprising one of various strains of the aforementioned fibre-producing species, under appropriate culture conditions, with one or more suitable electron donor components and one or more suitable electron acceptor components, examples of the latter including but not limited to oxygen. Electron acceptors could be any compound, but preferably, the electron acceptor is a soluble/dissolved electron acceptor, such as oxygen and/or nitrate. In one embodiment, it is provided that the electron acceptor is not a solid electron acceptor. A preferred electron acceptor is oxygen. After a time sufficient for adequate protein expression and culture growth, fibres and/or subfibre filaments can be isolated or extracted from the cells, using various techniques known in the art, and used as described elsewhere herein. Conductive fibres and/or subfibre filaments can also be produced and isolated in their soluble form using the native host or engineered microorganisms and assembled as conductive materials into for example electronic components and/or devices in vitro.

The invention also provides a method of using a bacterial cell belonging to the Desulfobulbaceae family, to prepare an electronic circuit component or electronic device, where that cell express one or more conductive fibres and/or subfibre filaments, which are conductive on the inside and non-conductive on the outside, and when a plurality of fibres and/or subfibre filaments are expressed, the fibres and/or subfibre filaments are arranged in parallel in the periplasmic space along the length and around the periphery of the cell or a chain of cells, wherein the outside of said chain of cells is non-conductive. Such a method can comprise providing at least one bacterial cell belonging to the Desulfobulbaceae family, for example a bacteria belonging to the Desulfobulbus genus expressing one or more conductive fibres and/or subfibre filaments, which are conductive on the inside and non-conductive on the outside, and when a plurality of fibres are expressed, the fibres and/or subfibre filaments are arranged in parallel in the periplasmic space along the length and around the periphery of the cell or a chain of cells, wherein the outside of said chain of cells is non-conductive; providing a culture medium conditioned for conductive fibre and/or subfibre filament production, such a medium comprising an electron acceptor; and incubating the bacterium or bacteria in the medium over a time sufficient for fibre formation and/or subfibre filament formation. As described above and illustrated more fully, below, in various embodiments of this invention, such proteinaceous conductive materials can be expressed by various members of the Desulfobulbus genus and/or Desulfobulbaceae family, or by suitable recombinant hosts. In such or certain other embodiments, one or more conductive fibres can be isolated from the medium. If so, such a method can also comprise in vitro circuit assembly. Manifestation of certain embodiments can comprise application of a voltage across one or more fibre and/or subfibre filaments components.

The invention is also directed to micro and nano-dimensioned electronic device structures, any of which can comprise a conductive circuit component comprising one or more conductive microbial fibres and/or subfibre filaments. Obtainable as described above, and in more detail, below, such components can be contacted with, adsorbed on or applied, connected and/or coupled to a suitable substrate material. A voltage source, alone or together with one or more suitably-dimensioned circuit components, as would be known in the art, can be connected with or coupled to one or more conductive fibres and/or subfibre filaments in the fabrication of a particular device structure.

### Bacterial cells

A main aspect of the present invention relates to a microorganism, in particular a bacterial cell belonging to the Desulfobulbaceae family, which comprises genetic material encoding parallel conductive fibres and/or subfibre filaments located in the periplasmic space along the length and around the periphery of the bacteria. When the cells grow (proliferate), they will tend to grow in a chain, thereby forming a chain of cells. In such a chain of cells, the conductive fibres and/or subfibre filaments are located around the periphery, and run in parallel along the length of the chain of cells, cf. drawings.

Thus, the bacterial cell is able to form a chain of cells upon growth, where the parallel conductive fibres and/or subfibre filaments are located in the periplasmic space along the length and around the periphery of the chain of cells. The invention therefore relates to a bacterial cell belonging to the Desulfobulbaceae family, which upon proliferation forms a chain of bacterial cells, said chain comprising a plurality of parallel conductive fibres and/or subfibre filaments located in the periplasmic space along the length and around the periphery of said chain of cells.

The invention also in one aspect relates to a chain of bacterial cells of the invention as defined herein. In this aspect, the invention provides a chain of bacterial cells belonging to the Desulfobulbaceae family comprising a plurality of parallel conductive fibres and/or subfibre filaments located in the periplasmic space along the length and around the periphery of said chain of cells, wherein the outside of said chain of cells is non-conductive.

The conductive fibres and/or subfibre filaments produced by bacterial cells or chain of cells belonging to the Desulfobulbaceae family of this invention may be used as conductive fibres and/or subfibre filaments within the cells or they can be extracted from the cells, and used in isolated (non-cellular) form.

In a preferred embodiment, the outside of the bacterial cell or chain of cells belonging to the Desulfobulbaceae family, is non-conductive, while the fibres and/or subfibre filaments located inside the cells are conductive. Thus, the conductive fibres and/or subfibre filaments are electrically insulated from the cell surroundings, for example the media or any material used to assemble an electronic device, which incorporate the conductive fibre and/or subfibre material of the invention. Furthermore, each individual conductive fibre and/or subfibre filament located within a cell or chain of cells of the invention preferably comprises an insulating layer, which insulates each conductive fibre from neighbouring parallel conductive fibres. This means that each conductive fibre in the bacterial cell can be used to conduct an individual current, which is separate and different from the currents conducted by parallel fibres of the same chain of cells.

The bacterial cell or in particular, the chain of bacterial cells belonging to the Desulfobulbaceae family of the invention comprises a plurality of individual conductive fibres, such as in the order of 2-40, such as 10-30, such as 15-30, for example 20-25 parallel conductive fibres located in the periplasmic space along the length and around the periphery of said one or more cells or chain of cells. In one particular preferred embodiment, bacterial cell or in particular, the chain of bacterial cells comprises 24 such parallel conductive fibres.

The diameter of each conductive fibre is within the nanometer scale. In one embodiment, the diameter of a fibre is 10-200 nm, such as 20-190, for example 30-180, such as 40-170, for example 50-160, such as 60-150, for example 70-140, such as 70-130, for example 80-120 nm, such as 85-115, for example 90-110 nm. In a preferred embodiment, the diameter is around 80-120 nm.

The length of each conductive fibre depends on the length of the chain of bacterial cell. The length of the chain of cells depends on the growth conditions for the cells, and the time allowed for growth. The length of the fibres can be manipulated in order to generate fibres of specific sizes depending on the intended use of the fibres. The chain of bacterial cells can be from µm to cm scale, for example from 1 µm to 10 cm or more. Thus, the length of a conductive fibre of the invention may range from about 3 µm (corresponding to one cell) to 10 cm or more, which corresponds to several ten thousands of cells. Thus, in one embodiment, the conductive fibres are 0.001-10 cm in length, for example 0.01-10 cm, such as 0.05-10 cm, for example 0.1-10 cm, such as 0.2-10, such as 0.3-10, such as 0.4-10, such as 0.5-10, such as 0.6-10, such as 0.7-10, such as 0.8-10, such as 0.9-10, for example 1-10 cm, or 2-9 cm, or 3-9, or 3-8 cm in length.

Analogously, a chain of bacterial cells belonging to the Desulfobulbaceae family of the present invention comprise preferably at least 10 bacterial cells, and may comprise up to several thousand cells, such up to 10,000, for example up to 20,000; 30,000; 40,000; 50,000; 60,000; 70,000; 80,000; 90,000; or 100,000 cells or more. In one embodiment, a chain of cells comprise of 100-200,000 bacterial cells, such as 10-150,000, or 10-100,000 or 10-50,000 cells, such as 20-1000, for example 30-900 cells, such as 40-800, such as 50-700, such as 60-800, such as 70-700, such as 80-700, such as 90-700, such as 100-700, such as 200-700, such as 300-700, such as 400-800, such as 400-700 cells.

The bacterial cell is in one embodiment a bacterial cell belonging to the Desulfobulbaceae family, for example a bacteria belonging to the Desulfobulbus genus. Thus, in one embodiment, the bacterial cell is selected from bacterial cells belonging to the Desulfobulbaceae family. In another embodiment, the bacterial cell is selected from Desulfobulbus genus. In one specific embodiment, the bacterial cell is selected from the group consisting of Desulfobulbus elongates, Desulfobulbus japonicas, Desulfobulbus mediterraneus, Desulfobulbus propionicus and Desulfobulbus rhabdoformis. In another specific embodiment, the bacterial cell is selected from the group consisting of Desulfobulbus japonicas, Desulfobulbus mediterraneus and Desulfobulbus rhabdoformis. In one other specific embodiment, the bacterial cell is Desulfobulbus elongates, Desulfobulbus japonicas, Desulfobulbus mediterraneus, Desulfobulbus propionicus or Desulfobulbus rhabdoformis. Also, the chain if cells may comprise a mixture of the bacterial cells mentioned herein above.

### Conductive electronic material

A main object of the present invention is to provide a conductive electronic material of the bacteria of the present invention, in particular a chain of such bacteria as explained herein above. A main aspect of the invention is therefore a conductive material of bacteria, belonging to the Desulfobulbaceae family, which produce conductive fibres and/or subfibre filaments, which are arranged in parallel inside the cell in the periphery of the cell membrane. The conductive electronic material comprises or consists of one or more conductive fibres of the chain of bacterial cells. The conductive fibres and/or subfibre filaments can be used as conductive fibres within the cells, or they can be extracted from the cells, and used in isolated (non-cellular) form.

The bacterial cells are characterized in that they comprise a plurality of conductive fibres and/or subfibre filaments, which are arranged in parallel in the periplasmic space along the length and around the periphery of the cells.

When the cells grow (proliferate), they will tend to grow in a chain, thereby forming a chain of cells. In such a chain of cells, the conductive fibres and/or subfibre filaments are located around the periphery, and run in parallel along the length of the chain of cells, cf. drawings.

In a preferred embodiment, the outside of the cells is non-conductive, while the fibres and/or subfibre filaments located inside the cells are conductive, i.e. the conductive fibres and subfibre filaments are insulated by the cell. Thus, the conductive electronic material of the invention is non-conductive on the outside of the cells. Furthermore, each individual conductive fibre preferably comprises an electrically insulating layer, which insulates each conductive fibre from neighbouring parallel conductive fibres. Thus, each conductive fibre and/or subfibre filaments of the conductive material of the present invention is also conductive on the inside, but non-conductive on the outside. This means that each conductive fibre in the bacterial cell can be used to conduct an individual current, which is separate and different from currents conducted by parallel fibres of the same chain of cells.

The conductive electronic material comprises or consists of a plurality of individual conductive fibres, such as in the order of 2-40, such as 10-30, such as 15-30, for example 20-25 parallel conductive fibres located in the periplasmic space along the length and around the periphery of the chain of cells. In one embodiment, the conductive electronic material comprises or consists of 10, 11, 12, 13, 14, 15, 16, 17, 18, 19, 20, 21, 22, 23, 24, 25, 26, 27, 28, 29 or 30 such parallel conductive fibres. In one particular preferred embodiment, the conductive electronic material comprises or consists of 17 such parallel conductive fibres. Thus, in one embodiment, the conductive electronic material is a conductive fibre, and in another embodiment, the conductive electronic material is a plurality of conductive fibres.

The diameter and length of each conductive fibre and/or subfibre filaments are described herein above, and may range from 10-200 nm, such as around 80-120 nm in diameter, and 0.0001-10 cm in length.

The length of the conductive fibres and/or subfibre filaments, which are comprised in the conductive electronic material of the invention, matches the ranges of the lengths of the bacterial chains or fibres. This includes a conductive electronic material of a chain of bacterial cells of the invention, wherein the chain comprise at least 10 bacterial cells, such as 100-50,000 bacterial cells or more.

However, a specific conductive material where two or more conductive materials of the present invention are assembled may be even longer. Such assemblies comprising multiple conductive materials are also within the scope of the present invention. This includes for example electronic devices comprising conductive materials of the present invention.

The present invention provides a conductive electronic material comprising or consisting of one or more conductive fibres of a chain of bacterial cells, belonging to the Desulfobulbaceae family, which comprise a plurality of said conductive fibres and/or subfibre filaments arranged in parallel in the periplasmic space along the length and around the periphery of said chain of cells, wherein the outside of said chain of cells is non-conductive. In one preferred embodiment, the bacterial cells are bacterial cells belonging to the *Desulfobulbaceae* family, for example a bacteria belonging to the Desulfobulbus genus. Thus, in one embodiment, the bacterial cells are selected from bacterial cells belonging to the Desulfobulbaceae family. In another embodiment, the bacterial cells are selected from Desulfobulbus genus. In one specific embodiment, the bacterial cells are selected from the group consisting of Desulfobulbus elongates, Desulfobulbus japonicas, Desulfobulbus mediterraneus, Desulfobulbus propionicus and Desulfobulbus rhabdoformis. In another specific embodiment, the bacterial cells are selected from the group consisting of Desulfobulbus japonicas, Desulfobulbus mediterraneus and Desulfobulbus rhabdoformis. In one other specific embodiment, the bacterial cells are Desulfobulbus elongates, Desulfobulbus japonicas, Desulfobulbus mediterraneus, Desulfobulbus propionicus or Desulfobulbus rhabdoformis. Also, the chain if cells may comprise a mixture of the bacterial cells mentioned herein above.

The conductive electronic material of the invention is produced by a bacterial cell of the invention, and depending on the downstream applications, the conductive electronic material is used while embedded in the chain of bacterial cells. However, in one embodiment, the conductive electronic material is extracted from the cells, such as from the chain of bacterial cells belonging to the Desulfobulbaceae family.

### Method of preparing a conductive electronic material

In one aspect, the present invention provides a method of preparing a conductive electronic material as defined herein above. This aspect of the invention provides a method of preparing a conductive electronic material consisting of conductive fibres and/or subfibre filaments, said method comprising
a) providing bacterial cells belonging to the Desulfobulbaceae family capable of forming a plurality of parallel conductive fibres and/or subfibre filaments located in the periplasmic space along the length and around the periphery of the cells, wherein the outside of said cells is non-conductive, and
b) incubating said bacterial cells belonging to the Desulfobulbaceae family, in a medium conditioned for conductive fibre formation and/or subfibre filament formation, said medium comprising an electron donor and an electron acceptor, in sufficient time to produce one or more chains of bacterial cells, each chain comprising a plurality of parallel conductive fibres and/or subfibre filaments located in the periplasmic space along the length and around the periphery of said chain of cells.

A culture medium is selected, which support proliferation of the cells, and which also supports formation of conductive fibres and/or subfibre filaments. The medium should comprise a suitable energy source, and any other required nutrients and additives. In particular, the medium should comprise an electron donor and an electron acceptor. For example, oxygen is used as an electron acceptor. The medium is in one embodiment a gradient culture.

The chain of bacterial cells belonging to the Desulfobulbaceae family produced in step b) may be any of the lengths described above; in one embodiment, the chain comprises at least 10 bacterial cells, but preferably, the chain comprises at least 100 or more. However, the length of the chain depends on the desired length of the conductive fibre material to be obtained by the process.

In one embodiment, the conductive electronic material, such as the one or more conductive fibre and/or subfibre filament, for example the plurality of parallel conductive fibres, subfibre filaments and/or chain of bacterial cells are isolated from the medium. In one embodiment, the chain of bacterial cells is isolated from the medium. In another embodiment, the conductive electronic material, such as the one or more conductive fibre and/or subfibre filaments, for example the plurality of parallel conductive fibres and/or subfibre filaments is isolated from the one or more chains of bacterial cells.

The bacterial cells used in this method of the invention are preferably a bacterial cell of the invention, as defined herein above. In one embodiment, the bacterial cells are bacterial cells belonging to the Desulfobulbaceae family, for example a bacteria belonging to the Desulfobulbus genus.

One advantage of the provided method for producing a conductive electronic material is that the method allows mass production of uniform organic microwires and/or cellular wires. The bacterial cells used in the method of the invention also allows the conductive material, such as microwires and/or cellular wires as well as electronic components and devices comprising the conductive material, to be produced by self-organizing, allowing for example the production of growth guided electronic circuits, components or devices.

The conductive material can be used as components of nano-scale electronics, cf. herein below.

### Use of a chain of bacterial cells as a conductive material

One aspect of the invention relates to the use of the cells of the invention, in particular bacterial cells belonging to the Desulfobulbaceae family, and preferably a chain of bacterial cells belonging to the Desulfobulbaceae family, as conductive materials. Thus, in one embodiment, the invention provides a use of a chain of bacterial cells comprising a plurality of parallel conductive fibres and/or subfibre filaments located in the periplasmic space along the length and around the periphery of said chain of cells, wherein the outside of said chain of cells is non-conductive, as a conductive material.

The specific nature and characteristics of the bacterial cells used are defined herein above, and in one embodiment, the bacterial cell belonging to the Desulfobulbaceae family is, for example a bacteria belonging to the Desulfobulbus genus.

In one embodiment, a voltage is applied across said bundle of conductive fibres and/or subfibre filaments.

### Electronic components and devices

The invention also provides electronic components and/or devices, which comprise a conductive material of the invention. Accordingly, the invention also provides electronic devices comprising one or more electronic components of the invention.

In one aspect, the invention provides an electronic component comprising
a) a conductive material comprising or consisting of one or more conductive fibres and/or subfibre filaments of a chain of bacterial cells belonging to the Desulfobulbaceae family, which comprise a plurality of said conductive fibres and/or subfibre filaments arranged in parallel in the periplasmic space along the length and around the periphery of said chain of cells, wherein the outside of said chain of cells is non-conductive, or
b) a conductive material prepared by
   i) providing bacterial cells belonging to the Desulfobulbaceae family capable of forming a plurality of parallel conductive fibres and/or subfibre filaments located in the periplasmic space along the length and around the periphery of the cells, wherein the outside of said cells is non-conductive, and
   ii) incubating said bacterial cells belonging to the Desulfobulbaceae family in a medium conditioned for bacterial proliferation and conductive fibre formation, said medium comprising an electron donor and an electron acceptor, in sufficient time to produce one or more chains of bacterial cells, each chain comprising a plurality of parallel conductive fibres and/or subfibre filaments located in the periplasmic space along the length and around the periphery of said chain of cells. In another aspect, the invention provides an electronic device comprising an electronic component as defined above.

In a further aspect, the invention provides an electronic device comprising
a) a conductive material comprising or consisting of one or more conductive fibres of a chain of bacterial cells belonging to the Desulfobulbaceae family, which comprise a plurality of said conductive fibres and/or subfibre filaments arranged in parallel in the periplasmic space along the length and around the periphery of said chain of cells, wherein the outside of said chain of cells is non-conductive, or
b) a conductive material prepared by
   i) providing bacterial cells belonging to the Desulfobulbaceae family, capable of forming a plurality of parallel conductive fibres and/or subfibre filaments located in the periplasmic space along the length and around the periphery of the cells, wherein the outside of said cells is non-conductive, and
   ii) incubating said bacterial cells in a medium conditioned for bacterial proliferation and conductive fibre formation and/or subfibre filament formation, said medium comprising an electron donor and an electron acceptor, in sufficient time to produce one or more chains of bacterial cells, each chain comprising a plurality of parallel conductive fibres and/or subfibre filaments located in the periplasmic space along the length and around the periphery of said chain of cells.

Specific embodiments of the conductive materials under a) and b) above are defined elsewhere herein, and also represent specific embodiments of the electronic components and devices of the present invention. For example, the bacterial cell is preferably a bacterial cell belonging to the Desulfobulbaceae family, for example a bacteria belonging to the Desulfobulbus genus.

An electronic component includes any physical entity suitable for use in electronic devices or systems, which entity comprise conductive material. Thus, an electronic component is any physical entity in an electronic device or system used to affect electrons or their associated fields in a desired manner consistent with the intended function of the electronic device or system. Components are generally intended to be connected together, for example by being soldered to a printed circuit board (PCB), to create an electronic circuit with a particular function (for example an amplifier, radio receiver, or oscillator). Components may be packaged singly or in more complex groups as integrated circuits. Some common electronic components are capacitors, inductors, resistors, diodes, transistors, etc. Electronic components are often categorized as active (e.g. transistors and thyristors) or passive (e.g. resistors and capacitors).

Thus, an electronic component is a basic electronic element that is available in a discrete form (a discrete device or discrete component), and normally has two or more electrical terminals (or leads). These leads connect, usually soldered to a printed circuit board, to create an electronic circuit (a discrete circuit) with a particular function (for example an amplifier, radio receiver, or oscillator). Basic electronic components may be packaged discretely, as arrays or networks of like components, or integrated inside of packages such as semiconductor integrated circuits, hybrid integrated circuits, or thick film devices. The following list illustrates examples of electronic components of the present invention:

**Table 1. List of electronic components as examples of components of the invention, which comprise an electronic conductive material of the invention.**

| Terminals and connectors | Description | Examples |
|---|---|---|
| Terminal | | |
| Connector | | |
| **Cable assemblies** | | |
| Cables with connectors or terminals at their ends | | Power cord |
| | | Patch cord |
| | | Test lead |
| **Switches** | Components that can pass current ("closed") or break the flow of current ("open"): | |
| | | |
| Switch | Manually operated switch. Electrical description: SPST, SPDT, DPST, DPDT, NPNT (general) | |
| | Technology: slide switches, toggle switches, rocker switches, rotary switches, pushbutton switches | |
| Keypad | Array of pushbutton switches | |
| DIP switch | Small array of switches for internal configuration settings | |
| Footswitch | Foot-operated switch | |
| Knife switch | Switch with unenclosed conductors | |
| Micro switch | Mechanically activated switch with snap action | |
| Limit switch | Mechanically activated switch to sense limit of motion | |
| Mercury switch | Switch sensing tilt | |
| Centrifugal switch | Switch sensing centrifugal force due to rate of rotation | |
| Relay | Electrically operated switch (mechanical, also see Solid State Relay below) | |
| Reed switch | Magnetically activated switch | |
| Thermostat | Thermally activated switch | |
| Humidistat | Humidity activated switch | |
| Circuit Breaker | Switch opened in response to excessive current: a resettable fuse | |
| **Resistors** | Pass current in proportion to voltage (Ohm's law). | |
| Resistor - | fixed value | |
| Power resistor - | larger to safely dissipate heat generated | |
| SIP or DIP resistor network - | array of resistors in one package | |
| Variable resistor | | |
| Rheostat | Two terminal variable resistor (often for high power) | |
| Potentiometer | Three terminal variable | |
| | resistor (variable voltage divider) | |
| Trim pot | Small potentiometer, usually for internal adjustments | |
| Heater | heating element | |
| Resistance wire, Nichrome wire | wire of high-resistance material, often used as heating element | |
| Thermistor | temperature-varied resistor | |
| Humistor | humidity-varied resistor | |
| Varistor, Voltage Dependent Resistor, MOV | Passes current when excessive voltage present | |
| **Protection devices** | Passive components that protect circuits from excessive currents or voltages | |
| Fuse | over-current protection, one time use | |
| Circuit Breaker | resettable fuse in the form of a mechanical switch | |
| Resettable fuse or PolySwitch | circuit breaker action using solid state device | |
| Ground-fault protection or residual-current device - | circuit breaker sensitive to mains currents passing to ground | |
| Metal oxide varistor (MOV), surge absorber, TVS - | Over-voltage protection. | |
| Inrush current limiter - | protection against initial Inrush current | |
| Gas discharge tube - | protection against high voltage surges | |
| Spark gap - | electrodes with a gap to arc over at a high voltage | |
| Lightning arrester - | spark gap used to protect against lightning strikes | |
| **Capacitors** | Capacitors store and release electrical charge. They are used for filtering power supply lines, tuning resonant circuits, and for blocking DC voltages while passing AC signals, among numerous other uses. | |
| Capacitor | fixed capacitance | Capacitor network (array) |
| Variable capacitor | adjustable capacitance | Tuning capacitor - variable capacitor for tuning a radio, oscillator, or tuned circuit |
| | | Trimmer capacitor - small variable capacitor usually for internal adjustments |
| Varicap diode | AC capacitance varies according to the DC voltage applied | |
| **Magnetic (inductive) devices** | Electrical components that use magnetism: | |
| Inductor, coil, choke | | |
| Variable inductor | | |
| Saturable Inductor | | |
| Transformer | | |
| Magnetic amplifier (toroid) | | |
| ferrite impedances, beads | | |
| Motor / Generator | | |
| Solenoid | | |
| speaker / microphone | | |
| **Networks** | Components that use more than one type of passive component | |
| RC network | forms an RC circuit, used in snubbers | |
| LC Network | forms an LC circuit, used in tuneable transformers and RFI filters | |
| **Piezoelectric devices, crystals, resonators** | Passive components that use piezoelectric effect | |
| Components that use the effect to generate or filter high frequencies | | Crystal - a ceramic crystal used to generate precise frequencies (See the Modules class below for complete oscillators) |
| | | Ceramic resonator - a ceramic crystal used to generate semi-precise frequencies |
| | | Ceramic filter - a ceramic crystal used to filter a band of frequencies such as in radio receivers |
| | | surface acoustic wave (SAW) filters |
| Components that use the effect as mechanical transducers. | | Ultrasonic motor - Electric motor that uses the piezoelectric effect |
| | | For piezo buzzers and microphones, see the Transducer class below |
| **Power sources** | Sources of electrical power | |
| Battery | acid- or alkali-based power supply | |
| Fuel cell | an electrochemical generator | |
| Power supply | usually a mains hook-up | |
| Photo voltaic device | generates electricity from light | |
| Thermo electric generator | generates electricity from temperature gradients | |
| Electrical generator | an electromechanical power source | |
| **Transducers, sensors, detectors** | Transducers generate physical effects when driven by an electrical signal, or vice-versa. | |
| | Sensors (detectors) are transducers that react to | |
| | environmental conditions by changing their electrical properties or generating an electrical signal. | |
| | The Transducers listed here are single electronic components (as opposed to complete assemblies), and are passive (see Semiconductors and Tubes for active ones). Only the most common ones are listed here. | |
| Audio (see also Piezoelectric devices) | | Loudspeaker - Magnetic or piezoelectric device to generate full audio |
| | | Buzzer - Magnetic or piezoelectric sounder to generate tones |
| Position, motion | | Linear variable differential transformer (LVDT) - Magnetic - detects linear position |
| | | Rotary encoder, Shaft Encoder - Optical, magnetic, resistive or switches - detects absolute or relative angle or rotational speed |
| | | Inclinometer - Capacitive - detects angle with respect to gravity |
| | | Motion sensor, Vibration sensor |
| | | Flow meter - detects flow in liquid or gas |
| Force, torque | | Strain gauge - Piezoelectric or resistive - detects squeezing, stretching, twisting |
| | | Accelerometer - Piezoelectric - detects acceleration, gravity |
| Thermal | | Thermocouple, thermopile - Wires that generate a voltage proportional to delta temperature |
| | | Thermistor - Resistor whose resistance changes with |
| | | temperature, up PTC or down NTC |
| | | Resistance Temperature Detector (RTD) - Wire whose resistance changes with temperature |
| | | Bolometer - Device for measuring the power of incident electromagnetic radiation |
| | | Thermal cutoff - Switch that is opened or closed when a set temperature is exceeded |
| Magnetic field (see also Hall Effect in semiconductors) | | Magnetometer, Gauss meter |
| Electromagnetic, light | | Photo resistor - Light dependent resistor (LDR) |
| **Semiconductors (Diodes; transistors; Integrated circuits and Optoelectronic devices)** | | |
| **Diodes** | Conduct electricity easily in one direction, among more specific behaviors. | |
| Standard Diode, Rectifier, Bridge Rectifier | | |
| Schottky Diode, Hot Carrier Diode - | super fast diode with lower forward voltage drop | |
| Zener Diode | Passes current in reverse direction to provide a constant voltage reference | |
| Transient Voltage Suppression Diode (TVS), Unipolar or Bipolar | used to absorb high-voltage spikes | |
| Varactor, Tuning diode, Varicap, Variable Capacitance Diode | A diode whose AC capacitance varies according to the DC voltage applied. | |
| Light Emitting Diode (LED) | A diode that emits light | |
| LASER Diode | A semiconductor laser | |
| Photodiode | Passes current in proportion to incident light | |
| Avalanche Photodiode Photodiode with internal gain | | |
| Solar Cell, photovoltaic cell, PV array or panel, produces power from light | | |
| Diode for Alternating Current (DIAC, Trigger Diode, SIDAC) - | Often used to trigger an SCR | |
| Constant current Diode | | |
| Peltier cooler | A semiconductor heat pump | |
| **Transistors** | Active components used for amplification. | |
| Bipolar transistors | | |
| | | Bipolar Junction Transistor (BJT, or simply "transistor") - NPN or PNP - Photo transistor - Amplified photodetector |
| | | Darlington transistor - NPN or PNP - Photo Darlington - Amplified photodetector |
| | | Sziklai pair (Compound transistor, complementary Darlington) |
| Field effect transistor (FET) | | |
| | | Junction Field Effect Transistor (JFET) - N-CHANNEL or P-CHANNEL |
| | | Metal Oxide Semiconductor FET (MOSFET) - N-CHANNEL or P-CHANNEL |
| | | MEtal Semiconductor FET (MESFET) |
| | | High Electron Mobility Transistor (HEMT) |
| Thyristors | | Silicon Controlled Rectifier (SCR) - Passes current only after triggered by a sufficient control voltage on its gate |
| | | TRIode for Alternating Current (TRIAC) - Bidirectional SCR |
| | | UniJunction Transistor (UJT) |
| | | Programmable UniJunction Transistor (PUT) |
| | | Static Induction Transistor/Thyristor (SIT, SITh) |
| Composite transistors | | Insulated Gate Bipolar Transistor (IGBT) |
| **Integrated circuits** | | |
| Digital | | |
| Analog | | |
| | | Hall effect sensor - Senses a magnetic field |
| | | Current sensor - Senses a current through it |
| **Optoelectronic devices** | | |
| Optoelectronics | | Opto-Isolator, Opto-Coupler, Photo-Coupler - Photodiode, BJT, JFET, SCR, TRIAC, Zero-crossing TRIAC, Open collector IC, CMOS IC, Solid State Relay (SSR) |
| | | Opto Switch, Opto Interrupter, Optical Switch, Optical Interrupter, Photo switch, Photo Interrupter |
| | | LED Display - Seven-segment display, Sixteen-segment display, Dot matrix display |
| **Display technologies** | | |
| Current | | |
| | | Filament lamp (indicator lamp) |
| | | Vacuum fluorescent display (VFD) (preformed characters, 7 segment, starburst) |
| | | Cathode ray tube (CRT) (dot matrix scan (e.g. computer monitor), radial scan (e.g. radar), arbitrary scan (e.g. oscilloscope)) (monochrome & colour) |
| | | LCD (preformed characters, dot matrix) (passive, TFT) (monochrome, colour) |
| | | Neon (individual, 7 segment display) |
| | | LED (individual, 7 segment display, starburst display, dot matrix) |
| | | Flap indicator (numeric, preprinted messages) |
| | | Plasma display (dot matrix) |
| Obsolete: | | Filament lamp 7 segment display (aka 'minitron') |
| | | Nixie Tube |
| | | Dekatron (aka glow transfer tube) |
| | | Magic eye tube indicator |
| | | Penetron (a 2 colour see-through CRT) |
| **Vacuum tubes (Valves)** | | |
| Based on current conduction through a vacuum (see Vacuum tube) | | Diode or Rectifier tube |
| Amplifying tubes | | Triode |
| | | Tetrode |
| | | Pentode |
| | | Hexode |
| | | Pentagrid |
| | | Octode |
| | | Microwave tubes: Klystron, Magnetron or Traveling-wave tube |
| Optical detectors or emitters | | Phototube or Photodiode - tube equivalent of semiconductor photodiode |
| | | Photomultiplier tube - Phototube with internal gain |
| | | Cathode ray tube (CRT) or Television picture tube |
| | | Vacuum fluorescent display (VFD) - Modern non-raster sort of small CRT display |
| | | Magic eye tube - Small CRT display used as a tuning meter (obsolete) |
| | | X-ray tube - Produces x-rays |
| **Discharge devices** | | |
| Gas discharge tube | | |
| Obsolete: | | Mercury arc rectifier |
| | | Voltage regulator tube |
| | | Nixie tube |
| | | Thyratron |
| | | Ignitron |
| **Antennas** | Antennas transmit or receive radio waves | |
| Elemental dipole | | |
| Yagi | | |
| Phased array | | |
| Loop antenna | | |
| Parabolic dish | | |
| Log-periodic dipole array | | |
| Biconical | | |
| Feedhorn | | |
| **Assemblies, modules** | Multiple electronic components assembled in a device that is in itself used as a component | |
| Oscillator | | |
| Display devices | | Liquid crystal display (LCD) |
| | | Digital voltmeters |
| Filter | | |
| **Prototyping aids** | | |
| Wire-wrap | | |
| Breadboard | | |
| **Mechanical accessories** | | |
| Enclosure (electrical) | | |
| Heat sink | | |
| Fan | | |
| **Other** | | |
| Printed circuit boards | | |
| Lamp | | |
| Waveguide | | |
| Memristor | | |
| Obsolete | | Carbon amplifier (see Carbon microphones used as amplifiers) |
| | | Carbon arc (negative resistance device) |
| | | Dynamo (historic rf generator) |

The electronic components and devices of the invention may be used in any type of electrical system, which requires the use of conductive materials. In one preferred embodiment, the electronic component and/or device is microstructured or nanostructured.

The conductive fibres and/or subfibre filaments of the invention may be used as a single strand wire. However, the fibres may also be used as a bundle of wires, a multistranded wire, i.e. as a cable. This cellular wire and/or cable may be used to carry electricity and telecommunications signals. Thus, wires and cables of the invention may be used in telephone and data cables, and as conductors in electric power transmission, and heating.

In particular, the conductive fibres, conductive material, components and devices of the invention are environmental friendly and non-toxic to human and animals, which is a significant advantage of these products. Thus, the conductive fibres, conductive material, components and devices of the invention can be used in edible and biodegradeable circuits and biosensors, for example for application in foods, and incorporation in the body of human and animals, as well as environmental sensor. The conductive fibres, conductive material, components and devices also provides soft and flexible electronics, which is advantageous in many applications, for example in devices for invasive medical use. Also miniaturization of biosensors based on chemioluminescense can be achieved by these conductive products of the invention.

The conductive fibres, conductive material, components and devices of the invention are also usable in connection with energy supply or electron exchange in biosensors or micro bioreactors (feeding or draining electrons is a "clean" way to drive reduction or oxidation processes).

The conductive fibres, conductive material, components and devices of the invention are also allows the production of devices for enhanced oxidative bioremediation of less mobile contaminants in subsurface matrix.

The products can also be used for remote control of biomineralization and microbial nanoparticle generation.

### Examples

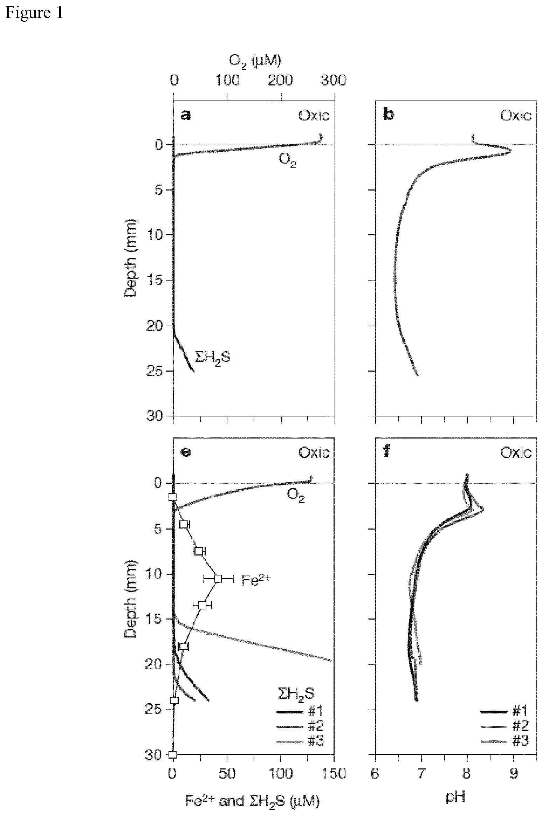

Figures 1-10 illustrates specific embodiments of the present invention.

Figure 1. Demonstration of electric grids in marine sediment. Porewater profiles of pH, hydrogen sulphide and oxygen in harbor sediment (a & b) and bay sediment (e & f) after about 3 weeks incubation with oxygen present in the overlying water. The vertical separation of oxygen and free hydrogen sulphide and the pH peak in the oxic zone could only be explained by the presence of centimeter long electric conductors transferring electrons from sulfide oxidation to oxygen reduction. Minimum current densities for the harbor and bay sediment were found to be 86 and 14 mA pr meter square, respectively (see Nielsen et al 2010 for details). Subsequent experiments with sediment confirmed the construction of an electric grid depending on vertical growth or migration of bacteria: Currents stopped if the conductors were cut by passing a thin wire through the sediment, and horizontal filters imbedded in the sediment could prevent the formation of an electric grid, only if the pore width was below typical bacterial cell diameter (0.8 micrometer).

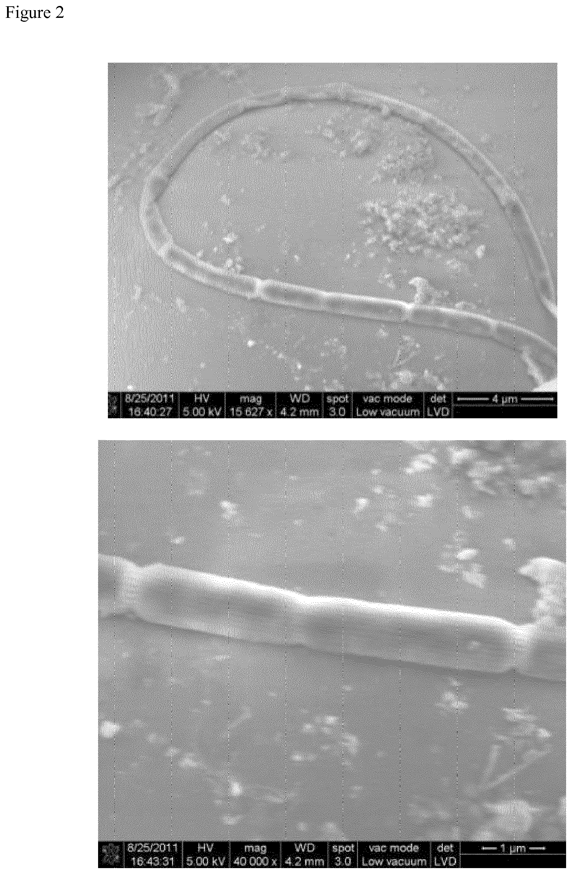

Figure 2. Observation of the cellular wire. Layers of washed glass beads were incubated with sulfidic sediment below and oxic sediment above, and electric currents were found to be established through the glass layer after 3 weeks. The beads were inspected with scanning electron microscopy (SEM) for possible conductive structures. Long filamentous bacteria were found to abound (a) and a closer view revealed uniform fibers or ridges running along the filaments with no interruptions at the cell junctions (b). These observations are in support that the bacteria serve as living electric cables holding a bunch of conductive fibers.

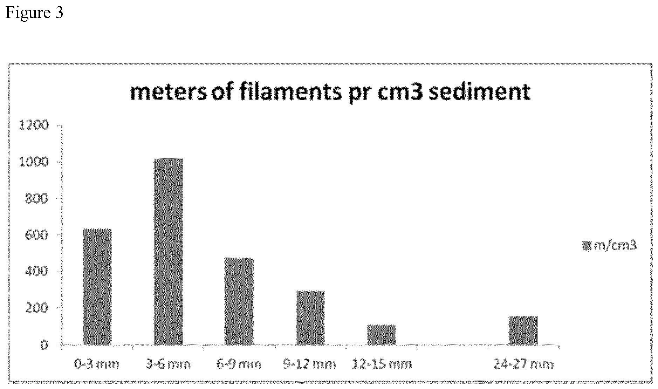

Figure 3. Abundance of filamentous bacteria in 2 week old "electric" sediment with oxygen penetration to 1.5 mm depth and detectable hydrogen sulfide is seen only at depths below 9 mm. Only filaments without sulfur and about 1 micrometer wide were recorded. Pretreatment and incubation of sulfidic sediment were performed as described in Nielsen et al. (2010), and filament density was measured by counting filament intercepts with search lines in subsamples inspected by light microscopy (Leica Apotome, 200 X, phase contrast). The distribution of bacterial filaments aligned very well with the electric zone read from the chemical profiles, and integrated with depth these microbial cellular wires amounted to about 6000 kilometers per square meter

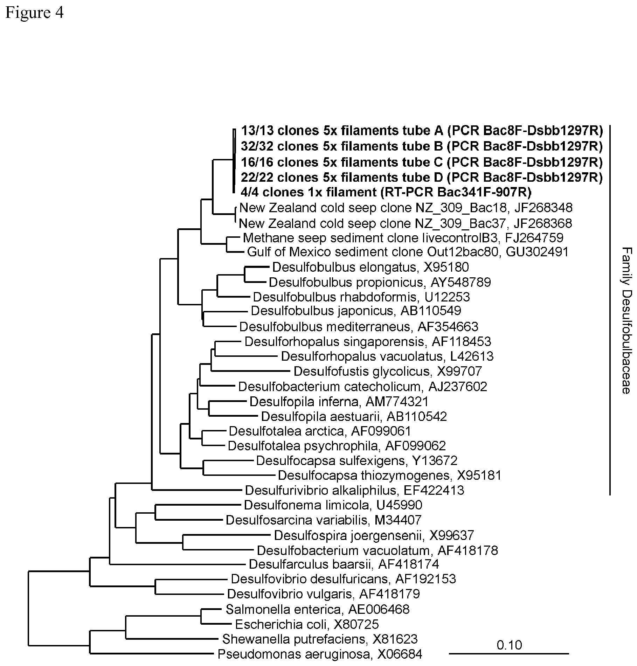

Figure 4. Sequencing of 16sDNA from bacteria in the electric zone showed high enrichment of several species of the family Desulfobulbaceae with Desulfobulbus propionicus as their closest, cultivated relative. As judged from the genetic distances, the detected electric zone species may appear to form a genus of their own

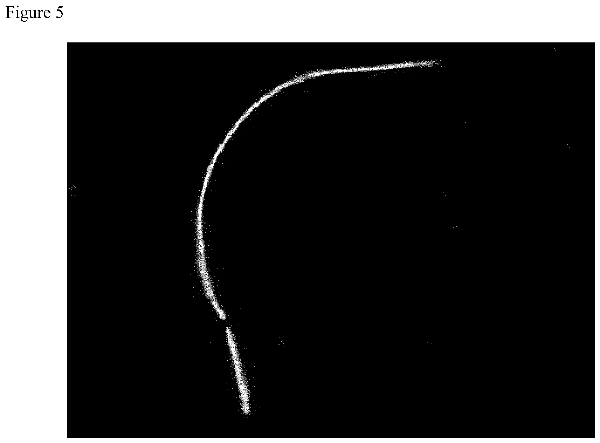

Figure 5. FISH (Fluorescent In Situ Hybridization) with a specific probe confirmed that the observed filaments (Figure 2 and 3) were indeed the Desulfobulbacea species found by sequencing (Figure 4).. A new probe targeting specifically the electric cable group of Desulfobulbaceae has now been developed as a tool for rapid screening and quantification in cultures and enrichments.

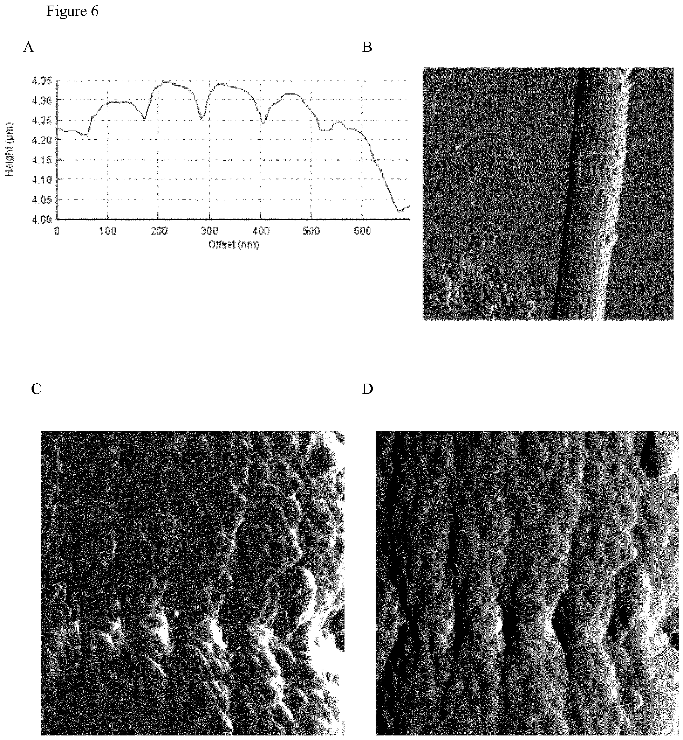

Figure 6. AFM (atomic force microscopy) investigation of the ridges running continuous from cell to cell of the bacterial filaments. A. Topographic transect across one filament showing that the ridges are well separated and have a uniform width of about 120 nanometer B. An AFM picture of a chain of cells forming a cable. At the cell-cell junction, a 700x700 nm square subsection is shown, which is enlarged in C (phase contrast) and D (amplitude).

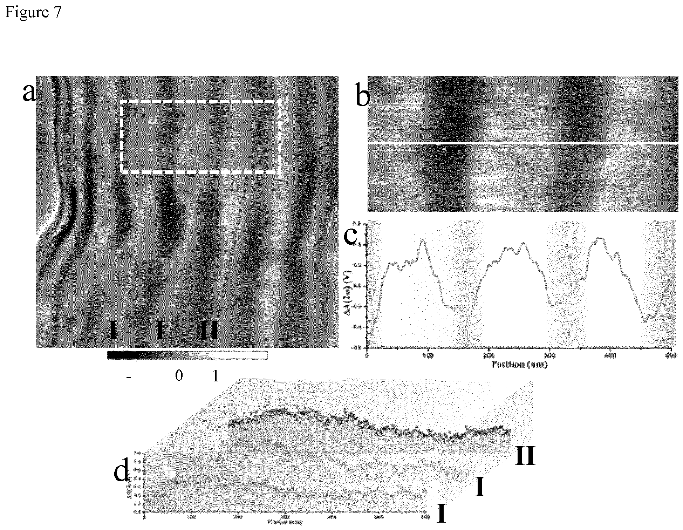

Figure 7. Investigation of the microbial/cellular cable with electrostatic force microscopy (EFM) revealed exceptionally high contrasting charge distribution for a biological material, suggesting the presence of electron conductors within the ridges. A: Charge distribution on the surface of a filament after all topographic effects have been filtered out. B and C: Close up of the insert in A showing the distinct separation of about 100 nm wide conductive bands with about 20 nm wide spacers in between. D: Longitudal records of along the lines marked in A showing continuity of charge distribution across the cell-cell junction.

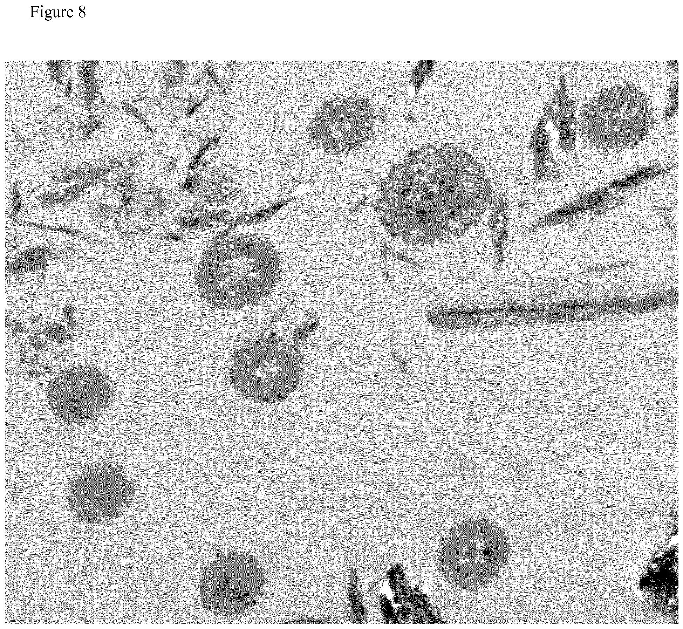

Figure 8. TEM (Transmission electron microscopy) image with crossection of 9 bacterial/cellular cables with sediment material around. They form two distinct groups probably representing species; small ones (∼ 400 nm Ø) having exactly 15 periferal ridges and large ones (∼ 700 nm Ø) having 17 ridges..

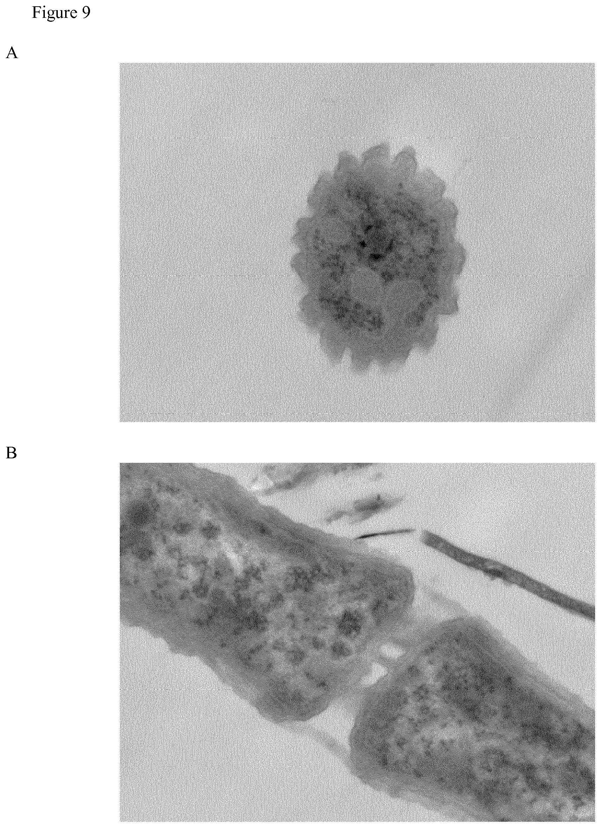

Figure 9. Transverse (A) and longitudal (B) TEM view of the bacterial/cellular cables. The ridges seemed to be composed of a uniform material separated from the cell interior by the cytoplasmic membrane and from the outside and adjacent ridges by the outer membrane. The material is thus situated in the periplasmic space and supposed to be the conductive fibers of the bacterial cable. An open space between cells was bridged by the fibers and the deflated outer membrane which therefore represented an emergent property of the whole, multicellular bacterium. The insulating effect of the outer membrane can explain why it so far have been impossible to record electronic properties of the filaments by applying metal electrodes on them.

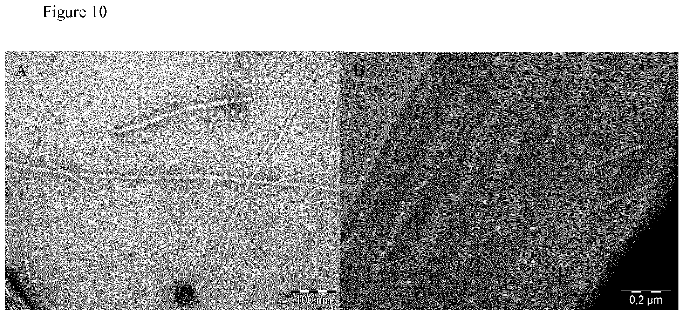

Figure 10. Negative stain EM on purified subfibre components showing individual ∼7 nm subfibre filaments and intertwined ∼7 nm subfibre filaments forming higher oligomers. B, Negative stain EM of a bacterial cable cell. Arrows indicate areas where it can be seen that ∼7 nm fragments run along/inside larger fibres having a size of approximately 80 nm.

## Claims

1. A conductive electronic material comprising or consisting of one or more conductive fibres and/or subfibre filaments of a chain of bacterial cells, wherein said chain of bacterial cells comprises a plurality of said conductive fibres and/or subfibre filaments arranged in parallel in the periplasmic space along the length and around the periphery of said chain of cells, wherein the outside of said chain of cells is non-conductive, wherein said bacterial cells belong to the Desulfobulbaceae family, and wherein said conductive electronic material comprises or consists of said chain of bacterial cells or is extracted from said chain of bacterial cells.

2. The conductive electronic material of claim 1, wherein each conductive fibre and/or subfibre filament comprise an insulating layer, which insulate each conductive fibre from neighbouring parallel conductive fibres.

3. The conductive electronic material of any of the preceding claims, wherein said bacterial cells form one or more chains of at least 10 bacterial cells, such as a chain of 100-50,000 bacterial cells or more.

4. The conductive electronic material of any of the preceding claims, wherein said conductive material comprises or consists of 2-40, such as 10-30, such as 15-30, for example 20-25, such as 24 parallel conductive fibres and/or subfibre filaments located in the periplasmic space along the length and around the periphery of said chain of cells.

5. The conductive electronic material of any of the preceding claims, wherein said bacterial cell belongs to the Desulfobulbus genus

6. The conductive electronic material of any of the preceding claims, wherein said conductive material is extracted from said chain of bacterial cells.

7. A method of preparing a conductive electronic material consisting of conductive fibres and/or subfibre filaments, said method comprising
a) providing bacterial cells belonging to the Desulfobulbaceae family and being capable of forming a plurality of parallel conductive fibres and/or subfibre filaments located in the periplasmic space along the length and around the periphery of the cells, wherein the outside of said cells is non-conductive, and
b) incubating said bacterial cells in a medium conditioned for bacterial proliferation and conductive fibre formation and/or subfibre filament formation, said medium comprising an electron donor and an electron acceptor, in sufficient time to produce one or more chains of bacterial cells, each chain comprising a plurality of parallel conductive fibres and/or subfibre filaments located in the periplasmic space along the length and around the periphery of said chain of cells.

8. The method of claim 7, wherein said medium is a gradient culture.

9. The method of any of claims 7-8, wherein said plurality of parallel conductive fibres, subfibre filaments and/or chain of bacterial cells is isolated from the medium.

10. The method of any of claims 7-9, wherein said bacterial cells belongs to Desulfobacterales, for example bacteria belonging to the Desulfobulbus genus.

11. Use of a chain of bacterial cells belonging to the Desulfobulbaceae family comprising a plurality of parallel conductive fibres and/or subfibre filaments located in the periplasmic space along the length and around the periphery of said chain of cells, wherein the outside of said chain of cells is non-conductive, as a conductive material.

12. The use according to claim 11, wherein said bacterial cells belongs to the Desulfobulbus genus.

13. An electronic device or component comprising
a) a conductive material as defined in any of claims 1 to 5, or
b) a conductive material produced as defined an any of claims 7 to 10

14. A chain of bacterial cells belonging to the Desulfobulbaceae family comprising a plurality of parallel conductive fibres and/or subfibre filaments located in the periplasmic space along the length and around the periphery of said chain of cells, wherein the outside of said chain of cells is non-conductive.

## Patentansprüche

1. Leitfähiges elektronisches Material, umfassend oder bestehend aus eine(r) oder mehrere(n) leitfähige(n) Fasern und/oder Subfaserfilamente(n) einer Kette von Bakterienzellen, wobei die Kette von Bakterienzellen eine Vielzahl der leitfähigen Fasern und/oder Subfaserfilamente umfasst, die parallel im periplasmatischen Raum entlang der Länge und um den Umfang der Zellenkette angeordnet ist, wobei die Außenseite der Zellenkette nichtleitfähig ist, wobei die Bakterienzellen der Familie Desulfobulbaceae angehören und wobei das leitfähige elektronische Material die Kette von Bakterienzellen umfasst oder daraus besteht oder aus der Kette von Bakterienzellen extrahiert ist.

2. Leitfähiges elektronisches Material nach Anspruch 1, wobei jede leitfähige Faser und/oder jedes leitfähige Subfaserfilament eine isolierende Schicht umfasst, die jede leitfähige Faser von benachbarten parallelen leitfähigen Fasern isoliert.

3. Leitfähiges elektronisches Material nach einem der vorhergehenden Ansprüche, wobei die Bakterienzellen eine oder mehrere Ketten von mindestens 10 Bakterienzellen bilden, wie eine Kette von 100-50.000 Bakterienzellen oder mehr.

4. Leitfähiges elektronisches Material nach einem der vorhergehenden Ansprüche, wobei das leitfähige Material 2-40, wie etwa 10-30, wie etwa 15-30, beispielsweise 20-25, wie etwa 24 parallele leitfähige Fasern und/oder Subfaserfilamente umfasst oder daraus besteht, die sich im periplasmatischen Raum entlang der Länge und um den Umfang der Zellenkette befinden.

5. Leitfähiges elektronisches Material nach einem der vorhergehenden Ansprüche, wobei die Bakterienzelle zur Gattung Desulfobulbus gehört.

6. Leitfähiges elektronisches Material nach einem der vorhergehenden Ansprüche, wobei das leitfähige Material aus der Kette von Bakterienzellen extrahiert wird.

7. Verfahren zur Herstellung eines leitfähigen elektronischen Materials, das aus leitfähigen Fasern und/oder Subfaserfilamenten besteht, wobei das Verfahren Folgendes umfasst:
a) Bereitstellen von Bakterienzellen, die der Familie Desulfobulbaceae angehören und in der Lage sind, eine Vielzahl von parallelen leitfähigen Fasern und/oder Subfaserfilamenten zu bilden, die sich im periplasmatischen Raum entlang der Länge und um den Umfang der Zellen befinden, wobei die Außenseite der Zellen nichtleitfähig ist, und
b) Inkubieren der Bakterienzellen in einem Medium, das für Bakterienproliferation und die Bildung leitfähiger Fasern und/oder Subfaserfilamente konditioniert ist, wobei das Medium einen Elektronendonator und einen Elektronenakzeptor umfasst, für eine Zeit, die ausreicht, um eine oder mehrere Ketten von Bakterienzellen zu erzeugen, wobei jede Kette eine Vielzahl von parallelen leitfähigen Fasern und/oder Subfaserfilamenten umfasst, die sich im periplasmatischen Raum entlang der Länge und um den Umfang der Zellenkette befinden.

8. Verfahren nach Anspruch 7, wobei das Medium eine Gradientenkultur ist.

9. Verfahren nach einem der Ansprüche 7-8, wobei die Vielzahl von parallelen leitfähigen Fasern, Subfaserfilamenten und/oder Ketten von Bakterienzellen aus dem Medium isoliert wird.

10. Verfahren nach einem der Ansprüche 7-9, wobei die Bakterienzellen Desulfobacterales angehören, beispielsweise Bakterien, die der Gattung Desulfobulbus angehören.

11. Verwendung einer Kette von Bakterienzellen, die der Familie Desulfobulbaceae angehören, die eine Vielzahl von parallelen leitfähigen Fasern und/oder Subfaserfilamenten umfasst, die sich im periplasmatischen Raum entlang der Länge und um den Umfang der Zellenkette befinden, wobei die Außenseite der Zellenkette nichtleitfähig ist, als leitfähiges Material.

12. Verwendung nach Anspruch 11, wobei die Bakterienzellen zur Gattung Desulfobulbus gehören.

13. Elektronisches Gerät oder Bauteil, umfassend:
a) ein leitfähiges Material nach einem der Ansprüche 1 bis 5 oder
b) ein leitfähiges Material, hergestellt nach einem der Ansprüche 7 bis 10.

14. Kette von Bakterienzellen, die der Familie Desulfobulbaceae angehören, die eine Vielzahl von parallelen leitfähigen Fasern und/oder Subfaserfilamenten umfasst, die sich im periplasmatischen Raum entlang der Länge und um den Umfang der Zellenkette befinden, wobei die Außenseite der Zellenkette nichtleitfähig ist.

## Revendications

1. Matériau électronique conducteur comprenant ou composé d'une ou de plusieurs fibres conductrices et/ou de filaments de sous-fibres d'une chaîne de cellules bactériennes, dans lequel ladite chaîne de cellules bactériennes comprend une pluralité desdites fibres conductrices et/ou de filaments de sous-fibres disposés en parallèle dans l'espace périplasmique le long de la longueur et autour de la périphérie de ladite chaîne de cellules, dans lequel l'extérieur de ladite chaîne de cellules est non conducteur, dans lequel lesdites cellules bactériennes appartiennent à la famille des Desulfobulbaceae, et dans lequel ledit matériau électronique conducteur comprend ou est composé d'une chaîne latérale de cellules bactériennes ou est extrait de ladite chaîne de cellules bactériennes.

2. Matériau électronique conducteur selon la revendication 1, dans lequel chaque fibre conductrice et/ou filament de sous-fibres comprend une couche isolante, qui isole chaque fibre conductrice des fibres conductrices parallèles avoisinantes.

3. Matériau électronique conducteur de l'une quelconque des revendications précédentes, dans lequel lesdites cellules bactériennes forment une ou plusieurs chaînes d'au moins 10 cellules bactériennes, telles qu'une chaîne de 100 à 50 000 cellules bactériennes ou plus.

4. Matériau électronique conducteur de l'une quelconque des revendications précédentes, dans lequel ledit matériau conducteur comprend ou est composé de 2 à 40, tel que 10 à 30, tel que 15 à 30, par ex., 20 à 25, tel que 24 fibres conductrices et/ou de filaments de sous-fibres parallèles localisés dans l'espace périplasmique le long de la longueur et autour de la périphérie de ladite chaîne de cellules.

5. Matériau électronique conducteur de l'une quelconque des revendications précédentes, dans lequel la cellule bactérienne appartient au genre Desulfobulbus.

6. Matériau électronique conducteur de l'une quelconque des revendications précédentes, dans lequel ledit matériau conducteur est extrait à partir de ladite chaîne de cellules bactériennes.

7. Méthode de préparation d'un matériau électronique conducteur composé de fibres conductrices et/ou de filaments de sous-fibres, ladite méthode comprenant
a) l'utilisation de cellules bactériennes appartenant à la famille des Desulfobulbaceae et qui peuvent former une pluralité de fibres conductrices et/ou de filaments de sous-fibres parallèles localisés dans l'espace périplasmique le long de la longueur et autour de la périphérie des cellules, dans laquelle l'extérieur desdites cellules est non conducteur, et
b) l'incubation desdites cellules bactériennes dans un milieu conditionné pour la prolifération bactérienne et la formation de fibres conductrices et/ou la formation de filaments de sous-fibres, ledit milieu comprenant un donneur d'électrons et un accepteur d'électrons, pendant une période suffisante pour produire une ou plusieurs chaînes de cellules bactériennes, chaque chaîne comprenant une pluralité de fibres conductrices et/ou de filaments de sous-fibres parallèles localisés dans l'espace périplasmique le long de la longueur et autour de la périphérie de ladite chaîne de cellules.

8. Méthode selon la revendication 7, dans laquelle ledit milieu est un gradient de culture.

9. Méthode selon l'une quelconque des revendications 7 à 8, dans laquelle ladite pluralité de fibres conductrices parallèles, de filaments de sous-fibres parallèles et/ou ladite chaîne de cellules bactériennes est isolée du milieu.

10. Méthode selon l'une quelconque des revendications 7 à 9, dans laquelle lesdites cellules bactériennes appartiennent aux Desulfobacterales, par exemple des bactéries appartenant au genre Desulfobulus.

11. Utilisation d'une chaîne de cellules bactériennes appartenant à la famille des Desulfobulbaceae comprenant une pluralité de fibres conductrices et/ou de filaments de sous-fibres parallèles localisés dans l'espace périplasmique le long de la longueur et autour de la périphérie de ladite chaîne de cellules, dans laquelle l'extérieur de ladite chaîne de cellules est non conducteur, comme matériau conducteur.

12. Utilisation selon la revendication 11, dans laquelle lesdites cellules bactériennes appartiennent au genre Desulfobulus.

13. Dispositif ou composant électronique comprenant
a) un matériau conducteur tel que défini dans l'une quelconque des revendications 1 à 5, ou
b) un matériau conducteur produit tel que défini dans l'une quelconque des revendications 7 à 10.

14. Chaîne de cellules bactériennes appartenant à la famille des Desulfobulbaceae comprenant une pluralité de fibres conductrices et/ou de filaments de sous-fibres parallèles localisés dans l'espace périplasmique le long de la longueur et autour de la périphérie de ladite chaîne de cellules, dans laquelle l'extérieur de ladite chaîne de cellules est non conducteur.
